# EUROPEAN PATENT SPECIFICATION

(11) **EP 1 401 642 B1**
(45) Date of publication and mention of the grant of the patent: **13.10.2004**
(21) Application number: 02733718.7
(22) Date of filing: 21.05.2002
(51) Int. Cl.: B32B 7/04, A61F 13/15

(54) **LAMINATE MADE OF FIBROUS LAYERS**
AUS FASERSCHICHTEN HERGESTELLTES LAMINAT
STRATIFIE FAIT DE COUCHES FIBREUSES

(30) Priority: 08.06.2001 SE 0102035
(43) Date of publication of application: 31.03.2004
(73) Proprietor: SCA Hygiene Products AB, 405 03 Göteborg (SE)
(72) Inventor: DREVIK, Solgun, S-435 35 Mölnlycke (SE); KVAMME, John, S-436 40 Askim (SE)
(74) Representative: Olsson, Stefan
(86) International application number: PCT/SE2002/000962
(87) International publication number: WO 2002/100632

(56) References cited:
- EP-A2- 1 153 737
- GB-A- 2 023 067
- GB-A- 2 335 627
- US-A- 4 392 862
- US-A- 5 383 870

## Description

### TECHNICAL FIELD

The present invention relates to a laminate made of fibrous layers for use in absorbent articles, such as sanitary towels, nappies or the like, the laminate including an outer layer made of non-woven fabric, which is in contact with the wearer during use of the article, and an inner layer, which two layers are interconnected in a first bonding pattern consisting of separate bonding points.

### BACKGROUND ART

Surface material for absorbent articles, such as sanitary towels, nappies or the like, means the layer which is located closest to the wearer and in contact with the body of the wearer during use of the article. A surface material must meet a great many functional requirements, some of which constitute contradictory requirements. The surface material is to feel soft and flexible to the wearer but must at the same time be strong so as to withstand wear. Another requirement is that it is to allow bodily fluid to pass through rapidly to the underlying absorbent body. The surface material is also to insulate the absorbent body from the wearer so that liquid which has been absorbed into the absorbent body should not be capable of back-wetting the wearer.

Conventional surface materials in sanitary towels and nappies are non-woven fabrics, which are commercially available in a great many variants. Depending on the selection of fibres included in the non-woven material and any addition of surface-active agents, such as wetting agents, the surface material is hydrophobic or hydrophilic. The degree of hydrophilicity can also be selected for controlling the liquid-permeability.

US patent 4,333,979 describes a surface material made of non-woven fabric, which is formed from thermoplastic fibres bonded in a pattern and is embossed so as to provide the surface material with increased thickness, softness and bulk at the same time as desired properties such as strength are said to be achieved. The non-woven material consists of what is known as spunbond with separate melt-bonding points lying closely together and with an applied embossing pattern in the form of separate embossings. The material in question is stated to be very effective as a surface material for disposable absorbent products, such as nappies, sanitary towels and the like. The material web is bonded by passing through a roller nip between a heated roller pair, one of which rollers consists of a pattern roller for forming said melt-bonding points. The production process also comprises permanent embossing of said embossing pattern in a roller nip formed by two heated, mutually matching embossing rollers. US patent 4,333,979 therefore describes a material web consisting of a single layer which, as mentioned above, is double-embossed, on the one hand for bonding the web and on the other hand for creating thickness and bulk. The material has very good strength properties, but at the expense of softness and flexibility, which are worse owing to the double embossing.

Perforated non-woven fabric, that is to say fabric in which holes have been made so as to increase the liquid flow capacity, has also been available for a long time. An example of a perforated non-woven fabric is described in EP 235 309. The perforated non-woven material according to said publication consists of what is known as spunlace material with a high content of hydrophobic fibres. In a spunlace process, holes are formed in the material by means of water jets, which are sprayed against the material at high pressure. The spunlace material constitutes one of two layers forming the surface material according to said publication and is intended to constitute the layer which is located closest to the wearer during use of the article. The aim is that the liquid be conducted through the holes into the underlying layer. The spunlace material has a higher content of hydrophobic fibres than the underlying layer in the laminate. The fibres in the upper spunlace layer consist of 70% hydrophobic fibres and 30% hydrophilic fibres; while the underlying material layer consists of equal parts of hydrophobic and hydrophilic fibres. The underlying layer therefore has the capacity to drain liquid from the upper layer.

One problem with the material described in EP 235 309, however, is that holes which are formed by water jets are irregular in terms of both size and shape and have fibres which protrude from the edges of the holes and into the holes. These protruding fibres reduce the hole areas, and liquid is moreover drawn into the material between the holes on account of capillary effects. The protruding fibre ends and the irregular shape and size of the holes considerably increase the risk of liquid remaining in the surface layer after wetting. As even a small quantity of liquid on the surface material is sufficient for this to feel wet or soiled to the wearer, this constitutes a major disadvantage of the material described in EP 235 309.

A similar material is described in EP 272 683. This publication also describes a surface material made of perforated non-woven fabric. Close to the holes, which are formed by perforating the non-woven material, there are relatively loose fibres, which are intended to function as ducts for transporting liquid down to an underlying non-woven layer of what is known as the meltblown type.

As long as the fibres in the perforated layer are arranged in such a manner that they conduct liquid down to an underlying layer, the surface material described functions. However, it is a well-known fact that a non-woven material consists of irregularly shaped fibres, which it is difficult to arrange in any particular direction. This means that fibres which are intended to transport liquid down to an underlying ply will also spread liquid over the surface of the non-woven material. Some of the liquid will for this reason remain in the surface material after wetting, and the article with the surface material in question will feel wet and unpleasant against the body of the wearer.

Another problem with the surface material described above is that it is difficult to obtain a well-defined hole size. From EP 409 535, for example, it is well-known that the size of the holes in the perforated material is critical for obtaining optimum liquid-permeability. In the case of a non-woven material which has some areas with a dense fibrous structure and other areas with a less dense structure, it is difficult to achieve the same hole size over the material. This is due to the fact that holes in denser fibrous areas are smaller because they are surrounded by more fibres.

Furthermore, perforated non-woven materials in such previously known surface materials have a relatively low tensile strength because the hole-manufacturing process described weakens the material. As it is important that the material has such a strength that there is no risk of it breaking during manufacture or when the article is in use, the low tensile strength is of course a major problem.

In EP 214 608, the holes are made in the non-woven material by means of hot needles, which heat the material to a temperature just below the melting point of the material. The holes made in the material therefore have a condensed portion of the fibrous material around each hole. The problems of different hole size and weakening of the material are thus eliminated to a certain extent. On the other hand, the problem of liquid being able to spread in the surface material and remain in the fibrous structure persists. The denser structure around the holes is also intended to draw liquid down into underlying layers, but there is an obvious risk of liquid remaining in the dense hydrophilic fibrous structure around the holes. There is also a risk of liquid spreading in the capillaries of the non-woven material. As the non-woven layer is in direct contact with the wearer, this represents a major disadvantage. Another problem is that, owing to the melting of portions around the holes, the surface material is stiff and uncomfortable for the wearer compared with material without condensed and partly melted portions around the holes.

In WO 9740793, it has been proposed, in conjunction with the hole-punching, to seal the area around each hole so as to reduce liquid spread in the lateral direction, each hole being surrounded by an essentially liquidtight edge. In this case, however, the problem remains that the material may feel relatively stiff and uncomfortable to the wearer compared with a similar material without these seals around the holes.

In addition to the use of non-woven fabrics, good results have been obtained in recent years using perforated films, that is to say plastic films with a large number of small holes which are designed so as to allow liquid through in one direction and prevent or reduce flow in the opposite direction. Good results in terms of low back-wetting have been achieved using such materials. An early example of such a film material is described in US 3,929,135. A later example of perforated plastic film is described in US 6,025,049. One disadvantage of perforated plastic films is that they have a plastic feel, which troubles many wearers who want softer material with a fibrous and textile feel.

It has proved difficult to provide all the desired properties of a surface material in a single layer, and it is therefore now common to have surface materials in the form of laminates consisting of two or more layers.

In order to achieve more rapid admission of liquid down into the absorbent core in an absorbent product, but also in order to create an insulating layer between the skin of the wearer and the absorbent core so as to prevent or at least reduce back-wetting, the surface material is now commonly combined with an underlying fibrous layer. An example of such a material combination is described in US 4,761,322. Another example is described in US 4,798,603, where a surface layer is supplemented by an underlying transport layer, said transport layer having a pore size which is smaller than the pore size in the surface layer. The latter publication states that such a material combination results in a higher liquid penetration rate and considerably lower back-wetting than a surface layer made only of, for example, spunbond. The surface layer in the absorbent article according to US 4,798,603 can be perforated for better liquid penetration.

In the case of material combinations consisting of two or more layers closest to the wearer, it is important that the layers make good contact with one another so that insulating gaps do not occur between the outer and inner layers, because such a gap would constitute an impediment to liquid passing from one layer to the other. It is therefore necessary to bond the two layers together to form a laminate. This can be effected using bonding agent or by thermal bonding, for example ultrasonic bonding.

The bonding together is often carried out at separate places in a bonding pattern, on the one hand in order not to constitute an impediment to liquid flow, and on the other hand in order that the laminate is not too stiff. Compared with a laminate without bonding pattern, however, the laminate is relatively stiff and feels hard and uncomfortable to a wearer.

It is clear from the above that there is still a real need to improve laminates for use as surface layers on absorbent articles, such as sanitary towels, nappies or the like.

### DISCLOSURE OF INVENTION

By means of the present invention, a laminate of the type mentioned in the introduction has been produced, which functions well and, compared with known solutions, is softer and more comfortable for the wearer.

The laminate according to the invention is characterized mainly in that holes are made at least through the outer layer in a hole pattern of separate holes, which hole pattern is denser with more holes per unit area than the number of bonding points per unit area in said first bonding pattern.

The purpose of the number of holes exceeding the bonding points between the outer and inner layers is that the holes are to soften the material.

According to one embodiment, the invention is characterized in that each of said holes is formed by arranging at least one slit through at least the outer layer and by pressing down an area forming the respective hole opening from the outside of the layer.

By virtue of the fact that the material penetration for the holes consists of slits, fibres in at least the outer layer are cut through at each slit, which results in this layer being more open, soft and flexible.

According to another embodiment, the laminate according to the invention is characterized in that each hole is formed by arranging two parallel slits delimiting the hole opening through at least the outer layer and by pressing down the rectangular area between the slits.

According to another embodiment, the invention is characterized in that the outer layer consists of non-woven fabric with bonding points arranged in a second bonding pattern, which second bonding pattern has fewer bonding points per unit area than the number of holes per unit area in said hole pattern.

According to another embodiment, the laminate according to the invention is characterized in that the inner layer consists of a non-woven containing at least polyester fibres.

Further suitable embodiments emerge from the claims which follow.

### BRIEF DESCRIPTION OF DRAWINGS

The invention will be described in greater detail below with reference to illustrative embodiments shown in the accompanying drawings, in which
- Figure 1: shows diagrammatically a plan view of a sanitary towel with a laminate according to the invention;
- Figure 2: shows a part of a cross section along the line II-II in Figure 1;
- Figure 3: shows diagrammatically a method of producing a laminate according to the invention;
- Figure 4: shows an embodiment of a hole in the laminate according to the invention;
- Figure 5: shows a second embodiment of a hole in the laminate according to the invention;
- Figure 6: shows diagrammatically a portion of an outer layer of an embodiment of the laminate according to the invention;
- Figure 7: shows diagrammatically the construction of an embodiment of a laminate according to the invention, and
- Figure 8: shows diagrammatically a second method of manufacturing a laminate according to the invention.

### MODE(S) FOR CARRYING OUT THE INVENTION

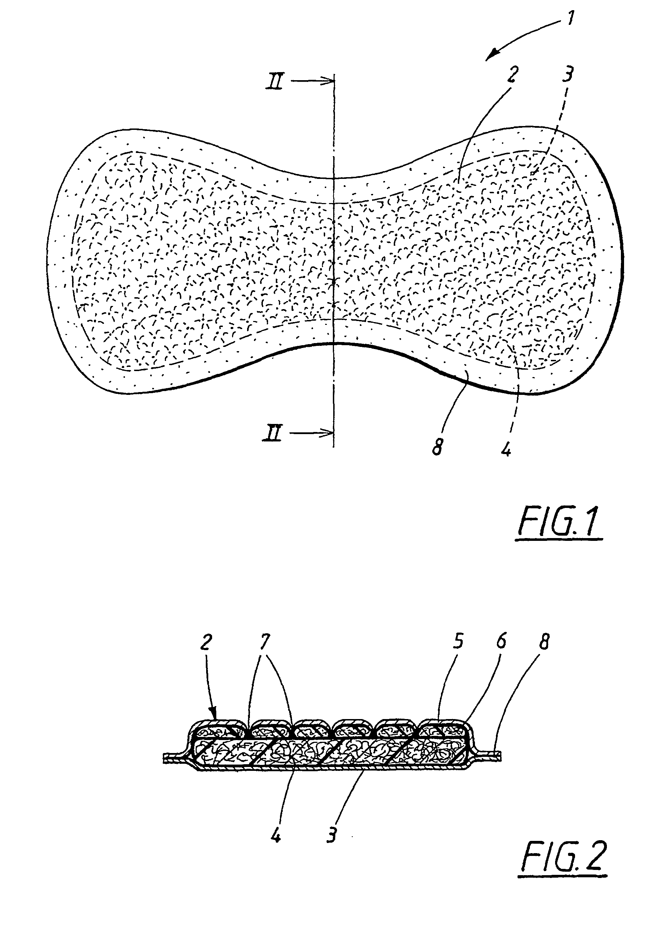

Figures 1 and 2 show a sanitary towel 1 with a liquid-permeable laminate 2, a liquidtight layer 3 in the form of a polyethylene film, and an absorbent body 4 arranged between the laminate and the liquidtight layer. The laminate consists of an outer layer 5 made of non-woven fabric, which is in contact with the wearer during use of the sanitary towel, and an inner layer 6 made of a fibrous material. The two layers 5, 6 making up the laminate according to the invention are interconnected in a first bonding pattern consisting of separate bonding points 7. This connection of the two layers making up the laminate will be described in greater detail below.

The expression bonding points is to be interpreted in such a way that the bonding points can have any shape and extent. For example, they can be in the form of a rhombus or a line.

The outer layer 5 in the laminate 2 extends a little way outside the inner layer 6 of the laminate around the entire periphery of the sanitary towel and is, outside the absorbent body 4, connected to the liquidtight plastic layer 3 along an edge portion 8.

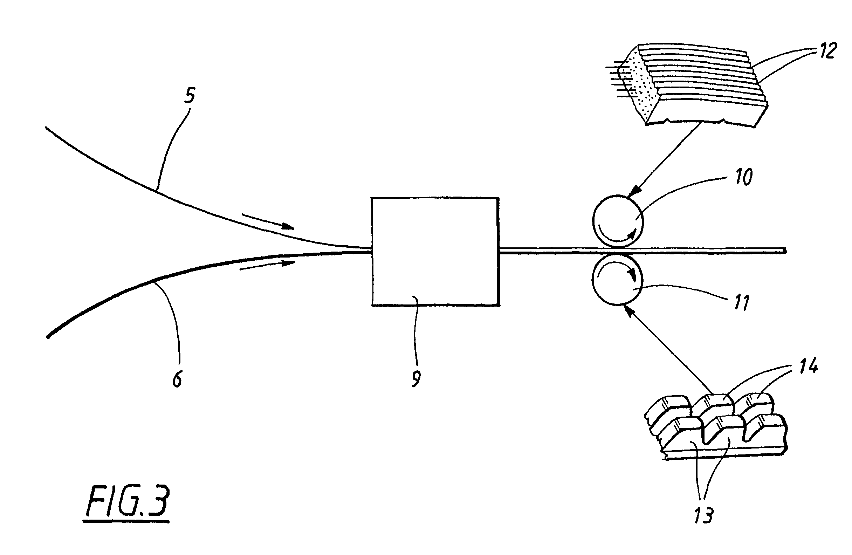

Figure 3 illustrates diagrammatically the production of a laminate 2 according to Figures 1 and 2. The outer layer 5 in the laminate is fed, together with the inner fibrous layer 6, to an ultrasound unit 9, which can be of conventional type and is not shown in greater detail. In the ultrasound unit, the two layers are bonded together in said bonding pattern consisting of separate bonding points.

The bonded-together laminate is then fed to a perforating unit in the form of two rollers 10 and 11, which are intended to cut separated longitudinal incisions in the laminate for forming holes. As can be seen from an enlarged portion, the roller 10 has parallel knives 12 which run all the way round and can be arranged on the periphery of the roller by, for example, milling. The counter-roller 11 has peripheral teeth 13 which engage in the material and, with their upper portions 14, press the laminate against the knives 12. A perforating arrangement of this type is described in greater detail in our GB patent 2 296 467.

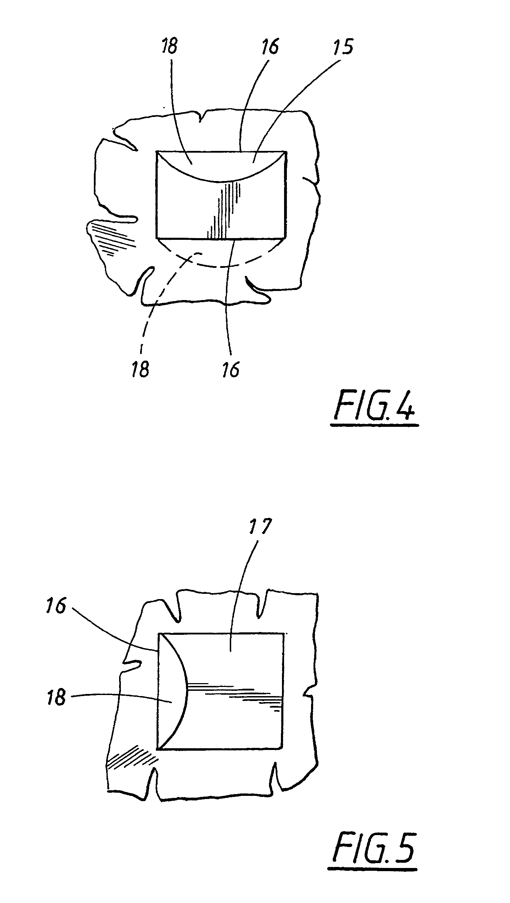

Figure 4 shows diagrammatically a piece of the laminate to illustrate how holes in the laminate are formed after it has been perforated by the perforating rollers 10, 11 shown in Figure 3. The hole, or rather the opening 15, in the laminate is delimited by two parallel incisions 16 cut by means of the knives 12. After the incisions 16 have been cut, a hammock-like portion is formed by pressing down the material between the slits 16 by means of the teeth 13 on the roller 11.

Figure 5 shows a hole which has been modified in relation to the hole according to Figure 4 and in which there is only one slit 16 and an adjacent area 17 of the laminate has been pressed by means of teeth of a different shape to those shown in Figure 3. The holes themselves in the laminate according to the embodiments shown are shaped like segments of a circle and have been designated by reference number 18 in Figures 4 and 5.

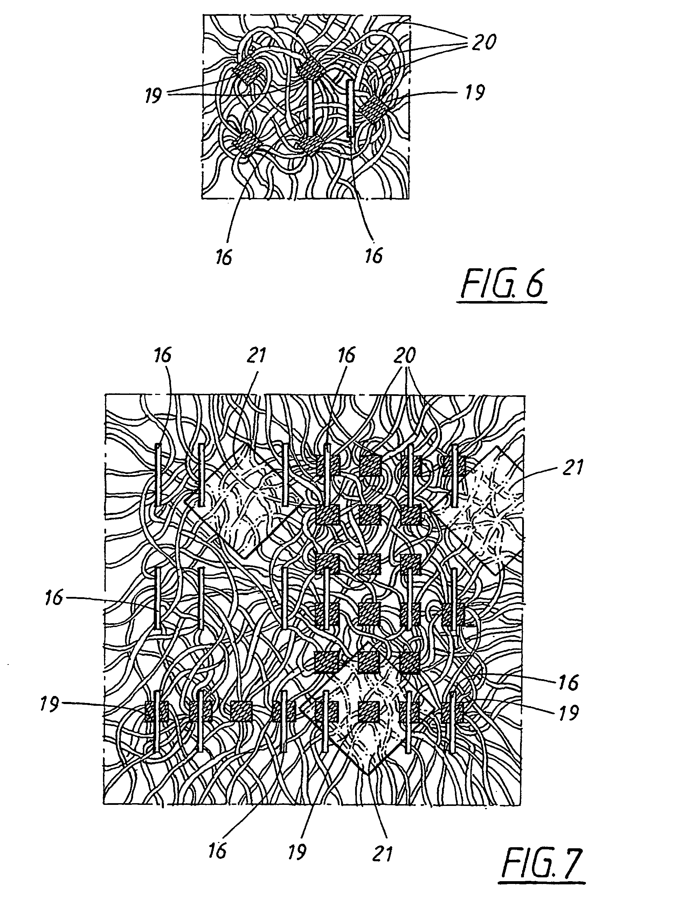

The outer layer in the laminate 2 according to the invention can consist of, for example, a spunbond non-woven. Figure 6 shows a portion of an outer layer 5 in the laminate according to the invention. According to one embodiment, the outer layer consists of a spunbond made of polypropylene fibres which, in this embodiment, are thermally bonded in a regular pattern of separate squares, which have been designated by reference number 19 in Figure 6. Figure 6 also shows the separated slits 16 described above. These are shown here in order to illustrate how individual fibres 20 in the spunbond material are cut through by the slits made. In Figure 6, which is diagrammatic, only two of the slits have been drawn. Owing to the arrangement of the slits, the material is very open and also soft and flexible.

The thermal bonding can be brought about by means of ultrasound. The pattern does not of course have to consist of a regular pattern of separate points, and other patterns and shapes are possible.

In Figure 7, which shows diagrammatically a portion of the whole laminate with inner and outer layers, the intention is again to illustrate how the slits 16 through the laminate open it up for forming flow holes but also for softening the material. In Figure 7, the same reference numbers as in Figure 6 have been used, reference number 19 indicating thermal bonding points in the bonding pattern of the outer spunbond layer. The slits through the material have been designated by reference number 16 and the spunbond fibres by reference number 20.

According to one embodiment, the inner layer consists of a carded synthetic wadding, comprising a mixture of PET fibres, PET/copolymer PET bonding fibres and PET/PP fibres. The wadding can be thermally bonded by means of hot air, what is known as through-air bonding. Alternative methods of bonding the wadding are by needling, thermobonding, bonding using water jets, or by bonding agents, such as latex. The two layers are interconnected by means of a first bonding pattern, which consists of separate bonding points. These have been indicated by reference number 21 in Figure 7. In the illustrative embodiment shown, bonding points of square shape have been selected, but other shapes are of course possible, for example rhombic or elongate bonding areas. These bonding points, which have according to one illustrative embodiment been brought about by means of ultrasound, are suitably larger in area than the thermal bonding points 19. As can be seen from Figure 7, the slits cut through fibres 20 and divide both the bonding points of the spunbond layer and the ultrasonically produced bonds 21 which bond together the two layers making up the laminate.

The size of the bonds 19 and 21 and the number of bonds per unit area in the respective bonding patterns for the outer layer 5 made of spunbond or other non-woven fabric and for the laminate can be varied as required by the intended properties with regard to durability and softness. The size of the slits 16 and the number of slits per unit area can also be varied to control the openness in the laminate and also to control the desired softness.

In the illustrative embodiment shown, bonding points of square shape have been selected, but other shapes are of course possible, for example rhombic or elongate bonding areas. When relatively large bonding points are used, such as the square ones shown, it may be suitable, in conjunction with the ultrasonic bonding, to knurl the square bonding points to form smaller separate parts. This is effected by, for example, dividing each bonding point up into four smaller squares which are separated from one another by elongate areas, the outer plane of which does not coincide with said smaller squares. By knurling, it is possible to avoid large smooth areas being formed at the bonding points. Such large smooth areas could otherwise result in drops of liquid, such as menstrual fluid, adhering to these large areas and not being drawn into the product.

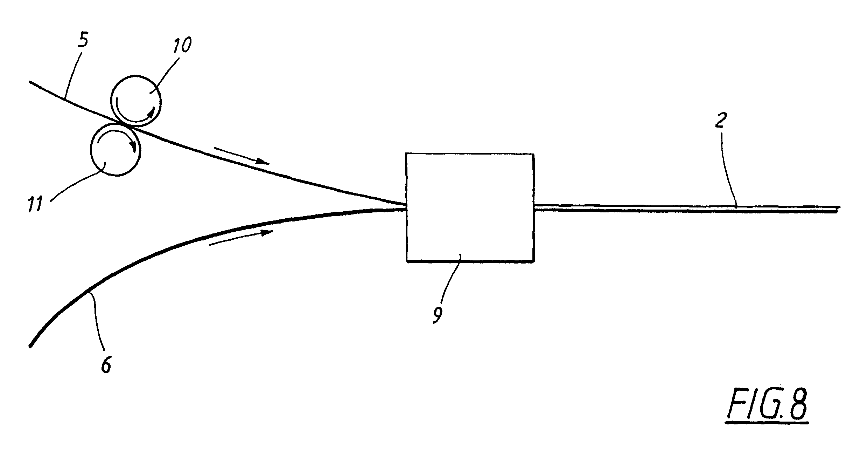

Figure 8 shows a method of manufacturing a laminate according to the invention, which has been modified slightly in relation to that according to Figure 3. The perforating unit in the form of perforating rollers 10, 11, described in connection with Figure 3 above, has in this case been arranged for perforation of only the spunbond material 5. Lamination of the outer perforated layer 5 and the inner fibrous layer 6 is performed subsequently in the ultrasound unit 9. The laminate 2 in the embodiment according to Figure 8 is therefore not as open as a laminate which has through-perforations, as described in connection with Figures 3-7 above. The open structure of the outer layer in the laminate, which open structure has been brought about by the perforations, the slits, through the spunbond layer results in this embodiment as well in a very soft surface material compared with previously known surface materials for use on absorbent articles.

The laminate according to the invention is not limited to the illustrative embodiments described above, but a number of modifications are possible within the scope of the patent claims below. For example, the number of holes per unit area can be greater than the number of thermal bonding points 19 per unit area in the spunbond layer, which produces a very soft and open material.

As described above, the outer layer 5 can consist of spunbond. Alternatively, the outer layer can consist of carded non-woven fabric. Spunbond and carded non-woven fabric can be bonded by hot air, that is to say what is known as through-air bonding, by needling, by thermobonding such as ultrasonic bonding, or by means of water jets.

In one illustrative embodiment shown, Fig. 4, each hole is formed by means of two adjacent parallel slits and by virtue of the material between the slits being pressed down so that hole openings which are essentially at right angles to the plane of the surface material are formed. This results in a very open surface material at the same time as softness is obtained by means of the slits. The holes in the outer layer can be made in another way. For example, the holes can consist of slits 16 in a plane outer layer, that is to say the outer layer is not pressed down in the areas between adjacent slits. The holes can also have any other shape. The essential feature is that fibres in the surface material are cut through so as to soften it.

## Claims

1. Laminate made of fibrous layers for use in absorbent articles, such as sanitary towels, nappies or the like, the laminate including an outer layer (5) made of non-woven fabric, which is in contact with the wearer during use of the article, and an inner layer (6), which two layers are interconnected in a first bonding pattern consisting of separate bonding points (7), **characterized in that** holes are made at least through the outer layer (5) in a hole pattern of separate holes, which hole pattern is denser with more holes per unit area than the number of bonding points (7, 21) per unit area in said first bonding pattern.

2. Laminate according to Claim 1, **characterized in that** each of said holes is formed by arranging at least one slit (16) through at least the outer layer (5) and by pressing down an area forming the respective hole opening from the outside of the layer.

3. Laminate according to Claim 1 or 2, **characterized in that** each hole is formed by arranging two parallel slits (16) delimiting the hole opening through at least the outer layer and by pressing down the rectangular area between the slits.

4. Laminate according to any one of the preceding claims, **characterized in that** the outer layer (5) consists of non-woven fabric with bonding points (19) arranged in a second bonding pattern, which second bonding pattern has fewer bonding points (19) per unit area than the number of holes per unit area in said hole pattern.

5. Laminate according to any one of the preceding claims, **characterized in that** the inner layer (6) consists of a non-woven containing at least polyester fibres.

6. Laminate according to Claim 5, **characterized in that** the inner layer (6) also contains bonding fibres in the form of PET/copolymer PET fibres.

7. Laminate according to any one of the preceding claims, **characterized in that** the outer layer (5) consists of a spunbond made of in the main polypropylene, with a weight per unit area in the order of 10-40 g/m², preferably 20-25 g/m².

8. Laminate according to any one of the preceding claims, **characterized in that** the outer and inner layers are thermally bonded to one another at said separate bonding points (7, 21) in the first bonding pattern.

9. Laminate according to Claim 8, **characterized in that** said thermal bonding is brought about by means of ultrasound.

10. Laminate according to any one of the preceding claims, **characterized in that** the outer layer (5) consists of a spunbond made of polypropylene, thermally bonded in a preferably regular second pattern of separate bonding areas with a total bonding area of in the order of 10-25%, preferably roughly 10-15%, in addition to which the number of bonding areas in said second bonding pattern is in the order of at least 10 and at most 55, preferably roughly 30-35 per cm².

11. Laminate according to any one of the preceding claims, **characterized in that** the number of bonding points (7, 21) in the first bonding pattern is in the order of at most 10 per cm².

12. Laminate according to any one of the preceding claims, **characterized in that** the inner layer (6) consists of a carded synthetic wadding, comprising a mixture of PET fibres, PET/copolymer PET bonding fibres and PET/PP fibres, which wadding is bonded by means of hot air.

13. Laminate according to any one of the preceding claims, **characterized in that** the inner layer (6) has a weight per unit area in the order of 30-70 g/m².

## Patentansprüche

1. Verbund aus faserigen Schichten zur Verwendung in Absorptionsgegenständen, wie beispielsweise Damenbinden, Windeln oder ähnlichem, wobei der Verbund eine Außenschicht (5) aus einem Vliesstoff, die während der Verwendung des Gegenstandes in Kontakt mit dem Träger steht und eine Innenschicht (6) umfasst, wobei die zwei Schichten in einem ersten Verbindungsmuster bestehend aus separaten Verbindungspunkten (7) miteinander verbunden sind, **dadurch gekennzeichnet, dass** wenigstens durch die Außenschicht (5) Löcher in einem Lochmuster aus separaten Löchern ausgebildet sind, wobei das Lochmuster mit mehr Löchern pro Flächeneinheit dichter als die Anzahl der Verbindungspunkte (7, 21) pro Flächeneinheit in dem ersten Verbindungsmuster ist.

2. Verbund nach Anspruch 1, **dadurch gekennzeichnet, dass** jedes der Löcher durch Anordnen wenigstens eines Schlitzes (16) durch wenigstens die Außenschicht (5) und durch Herunterdrücken eines Bereichs, der die entsprechende Lochöffnung bildet, von der Außenseite der Schicht ausgebildet ist.

3. Verbund nach Anspruch 1 oder 2, **dadurch gekennzeichnet, dass** jedes Loch durch Anordnen zweier die Lochöffnung begrenzender paralleler Schlitze (16) durch wenigstens die Außenschicht und durch Herunterdrücken des rechteckigen Bereichs zwischen den Schlitzen ausgebildet ist.

4. Verbund nach einem der vorhergehenden Ansprüche, **dadurch gekennzeichnet, dass** die Außenschicht (5) aus einem Vliesstoff mit in einem zweiten Verbindungsmuster angeordneten Verbindungspunkten (19) besteht, wobei das zweite Verbindungsmuster weniger Verbindungspunkte (19) pro Flächeneinheit aufweist als die Anzahl der Löcher pro Flächeneinheit in dem Lochmuster.

5. Verbund nach einem der vorhergehenden Ansprüche, **dadurch gekennzeichnet, dass** die Innenschicht (16) aus einem Vliesstoff besteht, der wenigstens Polyesterfasern enthält.

6. Verbund nach Anspruch 5, **dadurch gekennzeichnet, dass** die Innenschicht (6) auch Verbindungsfasern in der Form von PET/Copolymer PET Fasern enthält.

7. Verbund nach einem der vorhergehenden Ansprüche, **dadurch gekennzeichnet, dass** die Außenschicht (5) aus einem aus hauptsächlich Propylen gebildeten Spunbond mit einem Flächengewicht in der Größenordnung von 10-40 g/m² , vorzugsweise 20-25 g/m², gebildet ist.

8. Verbund nach einem der vorhergehenden Ansprüche, **dadurch gekennzeichnet, dass** die Außen- und Innenschicht an separaten Verbindungspunkten (7, 21) in dem ersten Verbindungsmuster thermisch miteinander verbunden sind.

9. Verbund nach Anspruch 8, **dadurch gekennzeichnet, dass** die thermische Verbindung mittels Ultraschall bewirkt wird.

10. Verbund nach einem der vorhergehenden Ansprüche, **dadurch gekennzeichnet, dass** die Außenschicht (5) aus einem aus Polypropylen gebildeten Spunbond besteht, das vorzugsweise in einem regelmäßigen zweiten Muster aus separaten Verbindungsbereichen mit einem gesamten Verbindungsbereich in der Größenordnung von 10-25%, vorzugsweise ungefähr 10-15%, thermisch verbunden ist, wobei zusätzlich dazu die Anzahl der Verbindungsbereiche in dem zweiten Verbindungsmuster in der Größenordnung von wenigstens 10 und höchstens 55, vorzugsweise ungefähr 30-35 pro cm² liegt.

11. Verbund nach einem der vorhergehenden Ansprüche, **dadurch gekennzeichnet, dass** die Anzahl der Verbindungspunkte (7, 21) in dem ersten Verbindungsmuster in der Größenordnung von höchstens 10 pro cm² liegt.

12. Verbund nach einem der vorhergehenden Ansprüche, **dadurch gekennzeichnet, dass** die Innenschicht (6) aus kardierter synthetischer Watte umfassend eine Mischung aus PET Fasern, PET/Copolymer PET Verbindungsfasern und PET/PP Fasern gebildet ist, wobei die Watte mittels heißer Luft verbunden ist.

13. Verbund nach einem der vorhergehenden Ansprüche, **dadurch gekennzeichnet, dass** die Innenschicht (6) ein Flächengewicht in der Größenordnung von 30-70 g/m² aufweist.

## Revendications

1. Lamellé constitué de couches de fibres destiné à être utilisé dans des articles absorbants tels que des serviettes hygiéniques, des couches-culottes ou des articles similaires, le lamellé comprenant une couche extérieure (5), constituée d'un textile non tissé, qui est en contact avec l'utilisateur pendant l'utilisation de l'article, et une couche intérieure (6), lesquelles deux couches sont interconnectées selon un premier motif de liaison constitué de points de liaison séparés (7), **caractérisé en ce que** des trous sont réalisés au moins dans la couche extérieure (5) selon un motif de trous constitué de trous séparés, lequel motif de trous est plus dense du fait qu'il comporte un plus grand nombre de trous par unité de surface que le nombre de points de liaison (7, 21) par unité de surface, dans ledit premier motif de liaison.

2. Lamellé selon la revendication 1, **caractérisé en ce que** chacun desdits trous est réalisé en agençant au moins une fente (16) dans au moins la couche extérieure (5) et en pressant en l'écrasant une surface formant l'ouverture respective du trou, à partir de l'extérieur de la couche.

3. Lamellé selon la revendication 1 ou 2, **caractérisé en ce que** chaque trou est réalisé en agençant deux fentes parallèles (16) délimitant l'ouverture du trou, dans au moins la couche extérieure et en pressant en l'écrasant la surface rectangulaire située entre les fentes.

4. Lamellé selon l'une quelconque des revendications qui précèdent, **caractérisé en ce que** la couche extérieure (5) est constituée d'un textile non tissé, comportant des points de liaison (19) agencés selon un deuxième motif de liaison, lequel deuxième motif de liaison comporte moins de points de liaison (19) par unité de surface que le nombre de trous par unité de surface dans ledit motif de trous.

5. Lamellé selon l'une quelconque des revendications qui précèdent, **caractérisé en ce que** la couche intérieure (6) est constituée d'un non-tissé contenant au moins des fibres polyester.

6. Lamellé selon la revendication 5, **caractérisé en ce que** la couche intérieure (6) contient également des fibres de liaison sous forme de fibres PET PET/copolymère.

7. Lamellé selon l'une quelconque des revendications qui précèdent, **caractérisé en ce que** la couche extérieure (5) est constituée d'un produit lié au filage constitué principalement de polypropylène, ayant un poids par unité de surface de l'ordre de 10 à 40 g/m², de préférence de 20 à 25 g/m².

8. Lamellé selon l'une quelconque des revendications qui précèdent, **caractérisé en ce que** les couches extérieure et intérieure sont liées thermiquement l'une à l'autre à l'emplacement desdits points de liaison (7, 21) dans le premier motif de liaison.

9. Lamellé selon la revendication 8, **caractérisé en ce que** ladite liaison thermique est effectuée au moyen d'ultrasons.

10. Lamellé selon l'une quelconque des revendications qui précèdent, **caractérisé en ce que** la couche extérieure (5) est constituée d'un produit lié au filage, fait de polypropylène, lié thermiquement selon un deuxième motif, de préférence régulier, de surfaces de liaison séparées, la surface de liaison totale étant de l'ordre de 10 à 25 %, de préférence, en gros de 10 à 15 %, en plus de quoi le nombre de surfaces de liaison présentes dans ledit deuxième motif de liaison est de l'ordre d'au moins 10 et d'au plus 55, de préférence, en gros, de 30 à 35/cm².

11. Lamellé selon l'une quelconque des revendications qui précèdent, **caractérisé en ce que** le nombre de points de liaison (7, 21), dans le premier motif de liaison, est de l'ordre d'au plus 10/cm².

12. Lamellé selon l'une quelconque des revendications qui précèdent, **caractérisé en ce que** la couche intérieure (6) est constituée d'une ouate synthétique cardée comprenant un mélange de fibres PET, de fibres de liaison PET PET/copolymère et de fibres PET/PP, laquelle ouate est liée au moyen d'air chaud.

13. Lamellé selon l'une quelconque des revendications qui précèdent, **caractérisé en ce que** la couche intérieure (6) présente un poids par unité de surface de l'ordre de 30 à 70 g/m².
